# EUROPEAN PATENT APPLICATION

(11) **EP 4 834 304 A1**
(43) Date of publication of application: **04.11.2026**
(21) Application number: 25786641.8
(22) Date of filing: 07.03.2025
(51) Int. Cl.: A61J 1/05, A61M 5/31

(54) **MEDICINE PACKAGING CONTAINER AND PREPARATION CONTAINER**

(30) Priority: 12.04.2024 JP 2024064436
(71) Applicant: Taisei Kako Co., Ltd., Kita-ku Osaka-shi Osaka 531-0072 (JP)
(72) Inventor: NAKAKUBO, Yutaro, Ibaraki-shi, Osaka 567-0054 (JP); UCHI, Shigenori, Ibaraki-shi, Osaka 567-0054 (JP); UMEZAKI, Masaya, Ibaraki-shi, Osaka 567-0054 (JP)
(74) Representative: DTS Patent- und Rechtsanwälte PartmbB
(86) International application number: PCT/JP2025/008348
(87) International publication number: WO 2025/215978

(57) **Abstract**

Provided is a pharmaceutical packaging container including a container body that is made of synthetic resin, has an elongated shape, is capable of containing medicine inside, and has openings for inlet and outlet for the medicine formed respectively at a distal end part and a proximal end part in an axial direction of the container body, and a wireless communication tag mounted to an outer surface of the container body, the container body including a tag mounting part for mounting the wireless communication tag thereto, the tag mounting part including a covering part that partially covers the wireless communication tag from the outside, and an exposing part that exposes the wireless communication tag outward from the outer surface of the container body.

## Description

### CROSS-REFERENCE TO RELATED APPLICATION

This application claims priority to Japanese Patent Application No. 2024-064436, the disclosure of which is incorporated herein by reference in its entirety.

### FIELD

The present invention relates to a pharmaceutical packaging container and a pharmaceutical container including a wireless communication tag capable of wireless communication.

### BACKGROUND

As an example of the above-mentioned pharmaceutical packaging container and pharmaceutical container, a medical infusion device with an embedded RFID tag is known (Patent Document 1). As shown in Fig. 17, a medical infusion device 901 includes a body 902 for containing a medical composition to be delivered, a hub portion 905 provided at an end of the body 902, and a cover 910 for covering the hub portion 905. A needle 908 is attached to the hub portion 905. The cover 910 includes an outer casing 920 and an inner casing 930. A ring-shaped RFID tag 940 is embedded between the outer casing 920 and the inner casing 930.

### CITATION LIST

### Patent Literature

Patent Literature 1: JP No. 2023-526093 T

### SUMMARY

### Technical Problem

However, in the conventional container described above, the RFID tag 940 is embedded in the cover 910, which is a different component from the body 902 that contains the medical composition. Therefore, if the cover 910 were to be illegally removed or replaced, the separation of the RFID tag 940 from the body 902 containing the medical composition could potentially compromise traceability. This container has significant environmental impact because it requires a large amount of resin to embed the RFID tag 940 into the resin using a mold.

Therefore, the present invention is to provide a pharmaceutical packaging container and a pharmaceutical container that can reliably ensure traceability and reduce environmental impact.

### Solution to Problem

A pharmaceutical packaging container including a container body that is made of synthetic resin, has an elongated shape, is capable of containing medicine inside, and has openings for inlet and outlet for the medicine formed respectively at a distal end part and a proximal end part in an axial direction of the container body, and a wireless communication tag mounted to an outer surface of the container body, the container body including a tag mounting part for mounting the wireless communication tag thereto, the tag mounting part including a covering part that partially covers the wireless communication tag from the outside, and an exposing part that exposes the wireless communication tag outward from the outer surface of the container body.

In the pharmaceutical packaging container of the present invention, the tag mounting part can include a plurality of the covering parts and a plurality of the exposing parts.

The pharmaceutical packaging container of the present invention can be configured such that the wireless communication tag includes a tag body that carries information, and an antenna part for allowing the tag body to communicate with an external device, the antenna part extending in a length direction that is a direction away from the tag body, the covering part configured to straddle the tag body or the antenna part in a direction intersecting the length direction.

The pharmaceutical packaging container of the present invention can be configured such that the wireless communication tag includes a tag body that carries information, and an antenna part for allowing the tag body to communicate with an external device, the antenna part extending in a length direction that is a direction away from the tag body, the plurality of covering parts configured to straddle the tag body or the antenna part in a direction intersecting the length direction.

The pharmaceutical packaging container of the present invention can be configured such that the container body has a barrel part having a cylinder shape extending in the axial direction for containing medicine between the distal end part and the proximal end part, the proximal end part includes a flange part that is formed around the opening at the proximal end part while protruding from the outer surface of the barrel part in a radially outward direction orthogonal to the axial direction, and the wireless communication tag is mounted to the flange part along a circumferential direction of the flange part.

A pharmaceutical container including a container body that is made of synthetic resin, and capable of containing a medical or pharmaceutical preparation inside, the container body including: a side wall part having a cylinder shape;
a shoulder part extending radially inward from a distal end part in an axial direction of the side wall part; a neck part connected to an end part on a radially inward side of the shoulder part and having a cylinder shape extending along the axial direction; and
a rim part that protrudes radially outward from the distal end part in the axial direction of the neck part, is formed in an annular shape around the axis, has an opening on the radially inner side, is configured to allow a plug member to be fitted into the opening and a cover member for covering the plug member to be engaged with an outer circumference, the pharmaceutical container further including: a wireless communication tag mounted to an outer surface of the container body, the container body including a tag mounting part to which the wireless communication tag is mounted, the tag mounting part including a covering part that partially covers the wireless communication tag from the outside and an exposing part that exposes the wireless communication tag outward from the outer surface of the container body.

### BRIEF DESCRIPTION OF DRAWINGS

Fig. 1 is a perspective view of a pharmaceutical packaging container according to one embodiment of the present invention.
Fig. 2 is a plan view of the pharmaceutical packaging container.
Fig. 3 is a cross-sectional view taken along a line III-III in Fig. 2.
Fig. 4 is a cross-sectional view taken along a line IV-IV in Fig. 2.
Fig. 5 is a cross-sectional view of a mold used in the manufacture of the pharmaceutical packaging container.
Fig. 6 is a cross-sectional view taken along a line VI-VI in Fig. 5.
Fig. 7 is a cross-sectional view taken along a line VII-VII in Fig. 5.
Fig. 8 is a perspective view of a pharmaceutical packaging container according to another embodiment of the present invention.
Fig. 9 is a plan view of the pharmaceutical packaging container.
Fig. 10 is a cross-sectional view taken along a line X-X in Fig. 9.
Fig. 11 is a cross-sectional view taken along a line XI-XI in Fig. 9.
Fig. 12 is a perspective view of a pharmaceutical container according to one embodiment of the present invention.
Fig. 13 is a plan view of the pharmaceutical container.
Fig. 14 is a cross-sectional view taken along a line XIV-XIV in Fig. 13.
Fig. 15 is a cross-sectional view taken along a line XV-XV in Fig. 13.
Fig. 16A is an enlarged plan view illustrating a pharmaceutical packaging container according to a modified example, showing a configuration in which an exposing part exposes most of a distal surface of a wireless communication tag.
Fig. 16B is an enlarged plan view illustrating a pharmaceutical packaging container according to a modified example, showing a configuration in which a covering part covers most of a distal surface of a wireless communication tag.
Fig. 16C is an enlarged plan view illustrating a pharmaceutical packaging container according to a modified example, showing a configuration in which a covering part covers one end part of the wireless communication tag in the circumferential direction from the outside, while a central part and the other end part of the wireless communication tag in the circumferential direction are exposed from the exposing part.

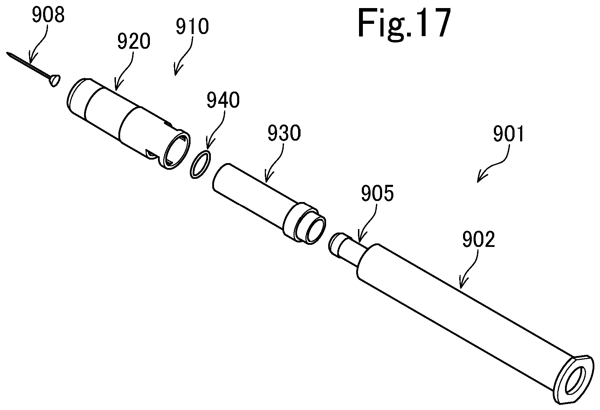

Fig. 17 is an exploded view of a conventional medical infusion device.

### DESCRIPTION OF EMBODIMENTS

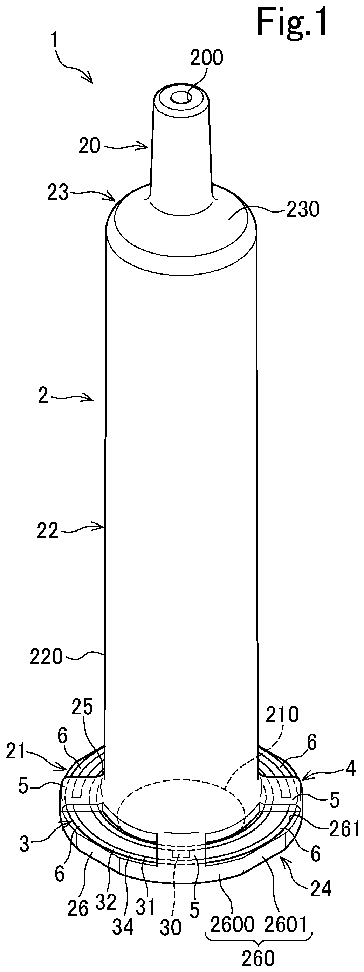

Hereinafter, a description will be hereinbelow given for a pharmaceutical packaging container according to one embodiment of the present invention with reference to Fig. 1 to Fig. 7.

As shown in Fig. 1, a pharmaceutical packaging container 1 according to this embodiment includes a container body 2 capable of containing medicine inside, and a wireless communication tag 3 mounted to the container body 2. This pharmaceutical packaging container 1 is a barrel for a syringe used to administer medicine.

The container body 2 contains, for example, medical liquid as medicine. The container body 2 has an elongated shape and has a distal end part 20 and a proximal end part 21 in an axial direction. Openings 200 and 210, which respectively serve as an inlet and an outlet for the medicine, are formed at both end parts 20 and 21 in the axial direction of the container body 2.

In this embodiment, the container body 2 has, in addition to the distal end part 20 and the proximal end part 21, a barrel part 22 that is disposed between the distal end part 20 and the proximal end part 21 and extends along the axial direction. The container body 2 has a shoulder part 23 disposed between the distal end part 20 and the barrel part 22. The container body 2 has a tag mounting part 4 for mounting the wireless communication tag 3 thereto.

The container body 2 can contain medicine other than the medical liquid, such as powdery medicine. In the pharmaceutical packaging container 1, the side where the distal end part 20 of the container body 2 is located, that is, the upper side in Fig. 1, is the "distal side," and the side where the proximal end part 21 of the container body 2 is located, that is, the lower side in Fig. 1, is the "proximal side." The "axial direction" of the container body 2 is a cylinder axis direction of the container body 2. The radial direction orthogonal to the axial direction is also simply referred to as "radially."

The container body 2 is made of synthetic resin, and is formed of a material that is, for example, transparent and can withstand the internal pressure applied when administering the medical liquid. Specifically, the material of the container body 2 is, for example, a resin containing cyclic olefins such as norbornene as repeating units. More specifically, the material of the container body 2 is, for example, a transparent resin such as COP (cycloolefin polymer), which is a homopolymer of cyclic olefins, or COC (cycloolefin copolymer), which is a copolymer of cyclic olefins and ethylene, etc.

The distal end part 20 is a part that discharges the medicine contained in the container body 2. The opening 200 is formed at the distal end part 20, which serves as the outlet for the medicine. The distal end part 20 has a substantially cylindrical shape. An outer diameter of the distal end part 20 decreases toward the distal side.

The barrel part 22 is a cylindrical body that contains the medicine. In this embodiment, the barrel part 22 has a cylindrical shape, but can have a square tube shape. The outer diameter of the barrel part 22 is smaller toward the distal side, but can be uniform. The outer diameter of the barrel part 22 is larger than the outer diameter of the distal end part 20 at any position in the axial direction.

The shoulder part 23 is a plate-shaped part that extends radially inward from the distal end edge of the barrel part 22 to the proximal end edge of the distal end part 20. The shoulder part 23 has an annular shape when viewed in the axial direction. Specifically, the shoulder part 23 has a ring shape. The outer surface 230 of the shoulder part 23 is an inclined surface that is inclined such that the inner diameter becomes smaller toward the distal side in the axial direction, but can be a flat surface.

The opening 210, which serves as an inlet for the medicine, is formed in the proximal end part 21. The proximal end part 21 projects radially outward from an outer surface 220 of the barrel part 22, in a direction orthogonal to the axial direction, and includes a flange part 24 formed around the opening 210 of the proximal end part 21.

The flange part 24 is a substantially plate-shaped part. The outer contour of the flange part 24 is located radially outward of the outer contour of the barrel part 22. This flange part 24 has a flange base part 25 provided on the proximal side of the barrel part 22. In this embodiment, the flange part 24 has a tag mounting part 4 in addition to the flange base part 25.

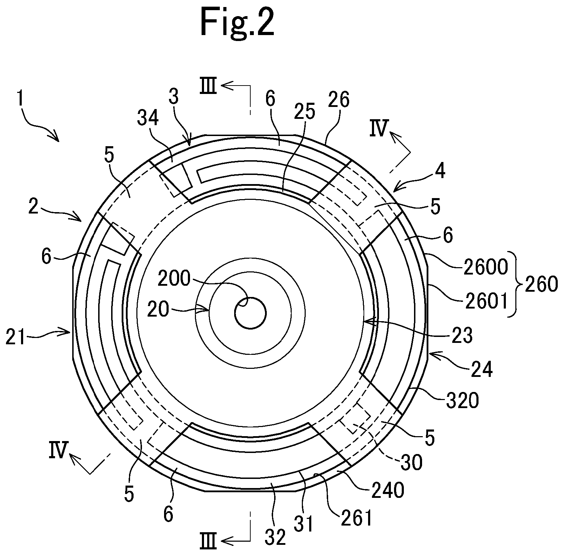

The flange base part 25 is an annular part extending from the proximal end edge of the barrel part 22. An opening is formed in the flange base part 25. The opening in the flange base part 25 forms the opening 210 in the proximal end part 21. In this embodiment, the flange base part 25 extends further toward the proximal side from the proximal end edge of the barrel part 22, and extends radially outward as shown in Fig. 2.

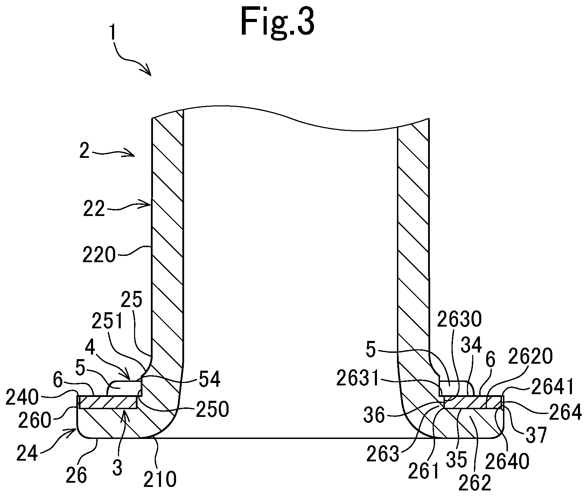

As shown in Fig. 3, the thickness of the flange base part 25 is greater than the thickness of the barrel part 22. The outer surface 250 of the flange base part 25, which faces the radially outer side, is located radially outward of the outer surface 220 of the barrel part 22. A distal surface 251 of the flange base part 25, which faces the distal side, is a curved surface that is recessed toward the proximal side. The distal surface 251 of the flange base part 25 can be a flat surface.

The tag mounting part 4 includes a covering part 5 that partially covers the wireless communication tag 3 from the outside, and an exposing part 6 that exposes the wireless communication tag 3 to the outside from the outer surface of the container body 2 (see Fig. 2). In this embodiment, the tag mounting part 4 is composed of, in addition to the covering part 5 and the exposing part 6, a flange body part 26 that extends radially outward from the radially outer edge of the flange base part 25.

The flange body part 26 is an annular part that extends radially outward from the radially outer edge of the flange base part 25. In this embodiment, the outer contour of the flange body part 26 has a substantially circumferential shape. The outer contour of the flange body part 26 has a substantially circumferential shape, combining an arcuate-shaped part and a straight part. Specifically, an outer edge 260 of the flange body part 26 is composed of four arcuate parts 2600 arranged at equal intervals in the circumferential direction, and four straight parts 2601 arranged between adjacent ones of the arcuate parts 2600. The outer edge 260 of the flange body part 26 can be a circular shape, or can be a polygonal shape such as a hexagon shape.

In this embodiment, the wireless communication tag 3 is disposed on the distal side of the flange body part 26. Specifically, a flange recess part 261 is disposed on the distal side of the flange body part 26, in which the wireless communication tag 3 is disposed. The flange body part 26 includes a flange support part 262 that supports the wireless communication tag 3 (see Fig. 3). The flange body part 26 includes an inner side wall part 263 disposed on the distal side of a part on the radially inner side of the flange support part 262, and an outer side wall part 264 disposed on the distal side of a part on the radially outer side of the flange support part 262.

The flange support part 262 is a part that supports the wireless communication tag 3 from the proximal side. A support surface 2620, which is a surface on the distal side of the flange support part 262, is a ring-shaped surface that is continuous in the circumferential direction.

The inner side wall part 263 is disposed on the radially inner side of the wireless communication tag 3. The inner side wall part 263 is an annular-shaped wall part that is continuous in the circumferential direction.

The outer side wall part 264 is disposed on the radially outer side of the wireless communication tag 3. The outer side wall part 264 is an annular-shaped wall part that is continuous in the circumferential direction. An edge 2642 on the radially outer side of the outer side wall part 264 of the flange body part 26 is continuous with an edge 2621 on the radially outer side of the flange support part 262.

The flange recess part 261 is a recess part that is continuous in the circumferential direction. The flange recess part 261 is defined by the support surface 2620 of the flange support part 262, an edge 2630 on the radially outer side of the inner side wall part 263, and an edge 2640 on the radially inner side of the outer side wall part 264.

The covering part 5 is a part that partially covers the wireless communication tag 3 from the distal side. In this embodiment, the covering part 5 is a part that covers the wireless communication tag 3 from the distal side partly in the circumferential direction of the flange part 24. Four covering parts 5 are provided (see Fig. 2). In this embodiment, the four covering parts 5 are arranged at intervals in the circumferential direction in the flange recess part 261 that is continuously provided in the circumferential direction. In the configuration where a plurality of the covering parts 5 are provided, it is preferable that at least one pair of covering parts 5 be arranged with the central axis of the flange part 24 therebetween. One covering part 5 can be provided, or four or more covering parts 5 can be provided. The circumferential dimension of the covering part 5 is uniform at any portions in the radial direction, but can be non-uniform. For example, the circumferential dimension of the covering part 5 can be larger toward the radially outer side.

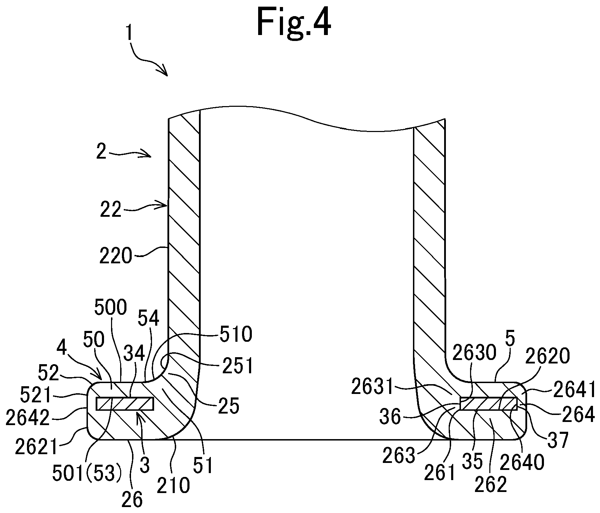

As shown in Fig. 4, the covering part 5 is provided continuously with the flange base part 25 and the flange body part 26. The covering part 5 is formed integrally with the flange base part 25 and the flange body part 26. In this embodiment, the covering part 5 includes a cover body part 50 located on the distal side of the wireless communication tag 3. The covering part 5 includes a cover inner edge part 51 located radially inward of the cover body part 50, and a cover outer edge part 52 located radially outward of the cover body part 50.

The thickness of the cover body part 50, and in this embodiment, the axial dimension of the cover body part 50, is substantially uniform. The distal surface 500, which is a surface facing the distal side of the cover body part 50, is a flat surface, but can be a curved surface.

The cover inner edge part 51 is a part that connects the cover body part 50 and the flange base part 25 to each other. The distal surface 510 of the cover inner edge part 51 facing the distal side is continuous with the distal surface 251 of the flange base part 25.

The cover outer edge part 52 is a part that extends toward the radially outer side from the cover body part 50. An edge 521 on the radially outer side of the cover outer edge part 52 is continuous with the edge 2642 on the radially outer side of the outer side wall part 264 of the flange body part 26.

The exposing part 6 is a part that exposes the wireless communication tag 3 outward from the outer surface 240 of the flange part 24 (see Fig. 3). In this embodiment, the exposing part 6 is a part that is open toward the distal side between the inner side wall part 263 and the outer side wall part 264 of the flange support part 262 to expose the wireless communication tag 3 toward the distal side therethrough. Four exposing parts 6 are provided, but one or a plurality of the exposing parts other than four can be provided (see Fig. 2).

The wireless communication tag 3 is, for example, a thin wireless communication tag, specifically, a substantially ring-shaped wireless communication tag. The wireless communication tag 3 is mounted to the outer surface of the container body 2. In this embodiment, the wireless communication tag 3 is mounted to the flange part 24 along the circumferential direction of the flange part 24. With the wireless communication tag 3 disposed in the flange recess part 261, its distal side is partially covered by the covering part 5.

The wireless communication tag 3 is an RFID tag that integrates both a UHF tag, which uses UHF band frequencies (enabling long-distance communication), and an NFC tag, which uses HF band frequencies, particularly a frequency of 13.56 MHz (enabling short-distance communication), into a single tag. The wireless communication tag 3 can be a passive type that transmits information written in a storage device in response to external access, or can be an active type that can spontaneously transmit information written in the storage device. The RFID tag can be an RFID tag consisting solely of the UHF tag or an RFID tag consisting solely of an NFC tag.

In this embodiment, the wireless communication tag 3 includes a tag body 30 that carries information and an antenna part 31 for allowing the tag body 30 to communicate with an external device. The wireless communication tag 3 includes a base part 32 where the tag body 30 and the antenna part 31 are provided.

The base part 32 is a substantially ring-shaped substrate or seal, etc. The outer contour of the base part 32 conforms to the outer contour of the flange body part 26. The outer edge 320 of the base part 32 has, for example, a circular shape. The outer edge 320 of the base part 32 can be composed of a plurality of arcuate parts arranged on the radially inner side of the arcuate part 2600 and a plurality of straight parts arranged on the radially inner side of the straight part 2601 of the flange body part 26.

The tag body 30 is a storage device for storing information, and is, for example, an IC chip. The antenna part 31 is a wireless communication device that wirelessly communicates information to be read from and written to the storage device. The antenna part 31 extends in the length direction, which is a direction away from the tag body 30. That is, in the antenna part 31, the proximal end is arranged on the tag body 30 side, and the distal end is located at a position away from the tag body 30, and in Fig. 2, at a position on the opposite side to the tag body 30 with the central axis of the flange part 24 therebetween. In this embodiment, the antenna part 31 extends along the direction in which the flange recess part 261 of the tag mounting part 4 extends. The length direction of the antenna part 31 extends along the circumferential direction of the wireless communication tag 3. Specifically, the antenna part 31 extends in a direction away from the tag body 30 in the circumferential direction, then extends in the radial direction, and then extends closer to the tag body 30 in the circumferential direction. More specifically, the antenna part 31 includes a part that extends clockwise away from the tag body 30 along the circumferential direction of the wireless communication tag 3 and counterclockwise toward the tag body 30 along this circumferential direction, and a part that extends counterclockwise away from the tag body 30 along the circumferential direction of the wireless communication tag 3 and clockwise toward the tag body 30 along this circumferential direction.

The covering part 5 is configured to straddle the tag body 30 or the antenna part 31 in a direction that intersects the length direction of the antenna part 31. In this embodiment, one covering part 5 is configured to straddle the tag body 30 and the distal side of the antenna part 31 in the radial direction, and three covering parts 5 are configured to respectively straddle the distal side of the antenna part 31 in the radial direction. The covering parts 5 are arranged to correspond respectively to the arcuate parts 2600, specifically, to the central positions in the circumferential direction of the arcuate parts 2600. The covering parts 5 can be configured to cover only a portion of the base part 32 where neither the tag body 30 nor the antenna part 31 is formed.

The wireless communication tag 3 is sandwiched between the covering part 5 and the flange support part 262 of the flange body part 26 in the axial direction (see Fig. 4). A distal surface 34, which is a main surface of the wireless communication tag 3 facing the distal side, is in contact with a covering surface 53, which is a surface of the covering part 5 facing the proximal side. In this embodiment, the covering surface 53 is composed of a proximal surface 501 which is a surface facing the proximal side of the cover body part 50. The proximal surface 35, which is a main surface of the wireless communication tag 3 facing the proximal side, is in contact with a support surface 2620 of the flange support part 262.

The wireless communication tag 3 is sandwiched between the inner side wall part 263 and the outer side wall part 264 of the flange body part 26 in the radial direction. An edge 36 on the radially inner side of the wireless communication tag 3 is in contact with the edge 2630 on the radially outer side of the inner side wall part 263. An edge 37 on the radially outer side of the wireless communication tag 3 is in contact with the edge 2640 on the radially inner side of the outer side wall part 264.

In this embodiment, the distal surface 34 of the wireless communication tag 3 is flush with the distal surface 2631, which is a surface of the inner side wall part 263 facing the distal side (see Fig. 3). The distal surface 34 of the wireless communication tag 3 is flush with the distal surface 2641, which is a surface of the outer side wall part 264 facing the distal side. A step is formed between the distal surface 54 of the covering part 5 and the distal surface 34 of the wireless communication tag 3. Specifically, the distal surface 54 of the covering part 5 is located on the distal side of the distal surface 34 of the wireless communication tag 3. The distal surface 54 of the covering part 5, which is a surface facing the distal side, and the distal surface 251 of the flange base part 25 are flush with each other (see Fig. 4).

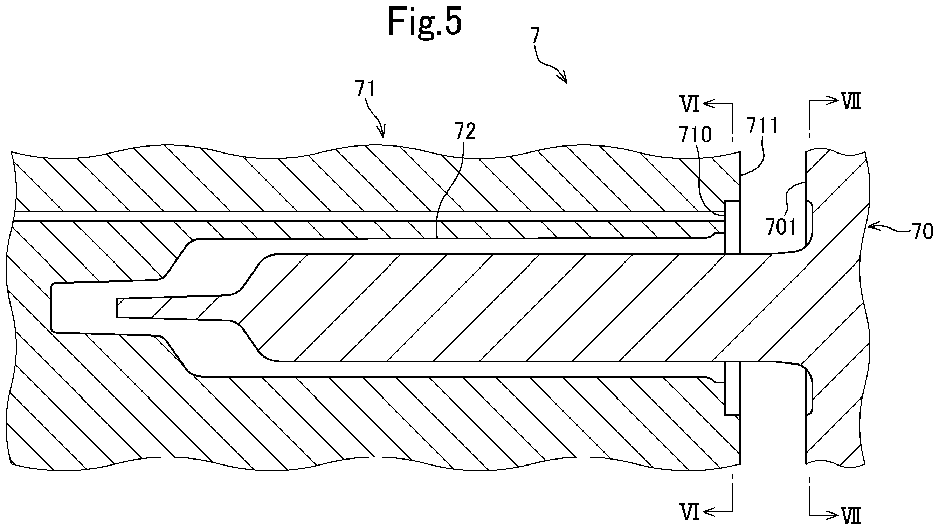

The thus described pharmaceutical packaging container 1 is manufactured by a single injection molding process using a mold 7 shown in Fig.5 to Fig. 7. As shown in Fig. 5, the mold 7 includes one core 70 and one cavity 71. Synthetic resin is injected into a space 72 formed between the core 70 and the cavity 71 in the mold 7 after clamping, with the wireless communication tag 3 placed inside. The mold 7 can be composed of a different number of parts, instead of the two parts 70 and 71 described above.

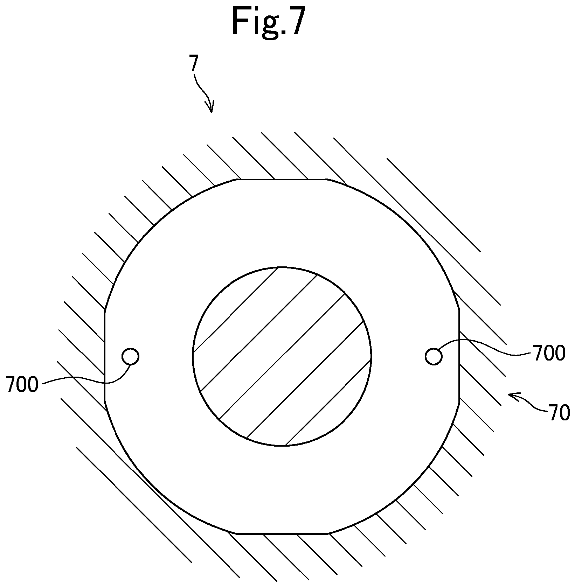

A gate 700 connected to an injection molding machine is formed in the core 70 (see Fig. 7). The gate 700 communicates with the space 72 in the mold 7 after clamping. The gate 700 is disposed at a position corresponding to the exposing part 6 of the tag mounting part 4 in this space 72. A pair of gates 700 are disposed in this embodiment, but instead one gate can be disposed, or three or more gates can be disposed.

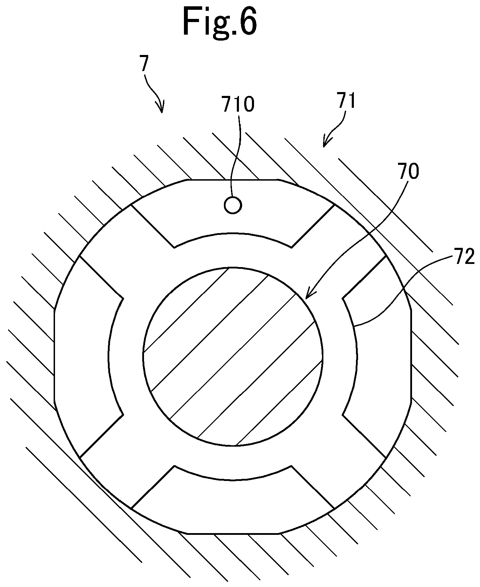

A suction hole 710 is formed in the cavity 71, through which the wireless communication tag 3 is suctioned and fixed at a position (see Fig. 6). The suction hole 710 communicates with the space 72 in the mold 7 after clamping (see Fig. 5). The suction hole 710 is disposed at a position corresponding to the exposing part 6 of the tag mounting part 4 in this space 72. The suction hole 710 is disposed so as not to overlap with any of the gates 700 in the axial direction. In this embodiment, only one suction hole 710 is provided, but instead a plurality of suction ports can be provided.

A description will be hereinafter given for a manufacturing method for the pharmaceutical packaging container 1. First, the wireless communication tag 3 is placed in the cavity 71, and then the wireless communication tag 3 is attached to the cavity 71 by suction through the suction hole 710 of the cavity 71. In this state, the cavity 71 is moved to a position where the proximal surface 711, which is a surface facing the proximal side of the cavity 71, abuts against the distal surface 701, which is a surface of the core 70 facing the distal side, thereby completing the mold clamping operation of the mold 7. In this way, the wireless communication tag 3 is suppressed from falling from the mold 7 by keeping the wireless communication tag 3 suctioned to the cavity 71 during the cavity 71 is moved.

Next, with the wireless communication tag 3 still suctioned to the cavity 71, molten resin is filled into the space 72 of the mold 7 through the gate 700. A part of this molten resin is made to flow into the space 72 of the mold 7 on the distal surface 34 side of the wireless communication tag 3, specifically flow to portions in the circumferential direction, thereby forming the covering parts 5 of the tag mounting part 4. The remaining molten resin is made to flow into other portions in the space 72 of the mold 7, thereby forming the parts other than the covering parts 5 of the container body 2, integrally with the covering parts 5. In this mold 7, the suction hole 710 is positioned so as not to overlap with the gate 700 in the axial direction. Therefore, it is possible to prevent the molten resin injected from the gate 700 from penetrating the wireless communication tag 3 and flowing into the suction hole 710.

The temperature of the container body 2 is lowered to harden the container body 2. Subsequently, suction through the suction hole 710 is stopped, and the cavity 71 is moved to open the mold 7, thereby obtaining the pharmaceutical packaging container 1.

In this manufacturing method, the wireless communication tag 3 remains suctioned to the mold 7 until the container body 2 hardens. Therefore, when the container body 2 is molded, the wireless communication tag 3 is fixed in position while being suctioned to the mold 7, allowing the wireless communication tag 3 to be accurately positioned in the container body 2. Alternatively, it can be configured such that after the container body 2 hardens, the cavity 71 is moved to open the mold 7 while the wireless communication tag 3 remains suctioned to the mold 7.

In the above-described pharmaceutical packaging container 1, the wireless communication tag 3 is disposed in the container body 2 that contains the medicine, thus ensuring traceability. Since the tag mounting part 4 includes the covering part 5 and the exposing part 6, the amount of resin used can be reduced by fixing the wireless communication tag 3 with the covering part 5 and exposing the wireless communication tag 3 with the exposing part 6, thereby reducing the environmental impact.

In the pharmaceutical packaging container 1 of this embodiment, the wireless communication tag 3 is securely fixed with a plurality of the covering parts 5, and the amount of resin used can be reduced by providing the plurality of exposing parts 6.

In the pharmaceutical packaging container 1 of this embodiment, the covering parts 5 each straddle the outside of the wireless communication tag 3, thereby securely fixing the wireless communication tag 3, and the covering parts 5 extend in a direction that intersects the length direction of the antenna part 31. That is, since the covering parts 5 partially cover the wireless communication tag 3 in the length direction of the antenna part 31, the amount of resin used when manufacturing the pharmaceutical packaging container 1 can be reduced.

In the pharmaceutical packaging container 1 of this embodiment, the wireless communication tag 3 is mounted to the flange part 24 along the circumferential direction of the flange part 24, thereby increasing the circumferential size of the wireless communication tag 3. This ensures that the communication range of the wireless communication tag 3 can be maintained. By providing the wireless communication tag 3 on the flange part 24, the wireless communication tag 3 does not hinder the handling of the pharmaceutical packaging container 1. The visibility of the medicine contained in the container body 2 is not impaired.

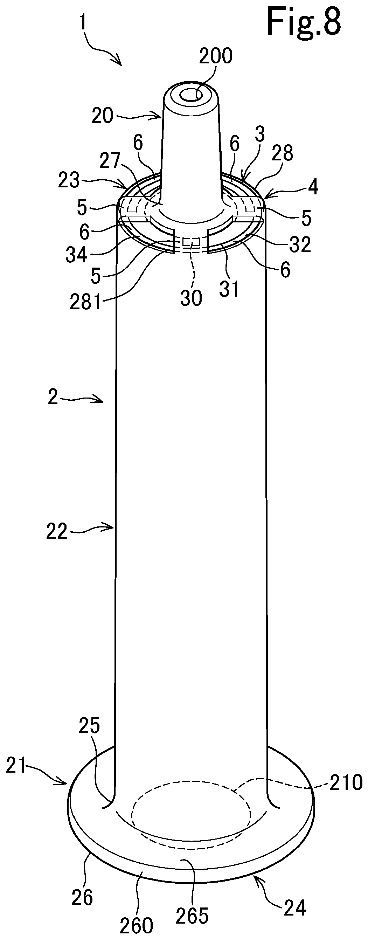

In the above embodiment, the wireless communication tag 3 is disposed on the distal side of the flange part 24, but the wireless communication tag 3 can be disposed on the proximal side of the flange part 24. Although the flange part 24 includes the tag mounting part 4, another part of the container body 2 can provide the tag mounting part 4. For example, the barrel part 22 of the container body 2 can be configured to have the tag mounting part 4, and have the wireless communication tag 3 disposed along the outer surface 220 of the barrel part 22. Alternatively, it can be configured such that the shoulder part 23 of the container body 2 has the tag mounting part 4, and the wireless communication tag 3 is disposed on the distal side of the shoulder part 23. A description will be hereinbelow given for an example of a configuration in which the shoulder part 23 has the tag mounting part 4 with reference to Fig. 8 to Fig. 11.

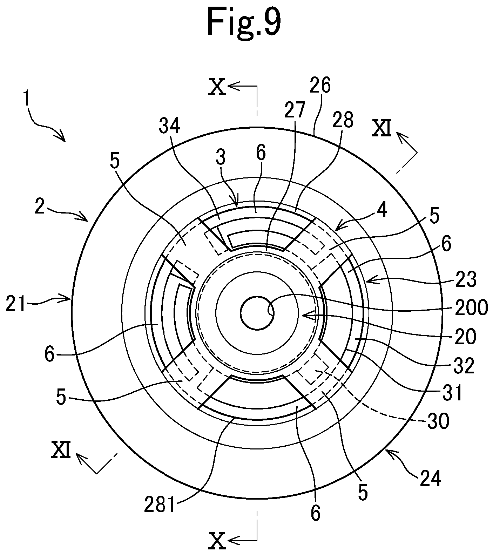

As shown in Fig. 8 and Fig. 9, the shoulder part 23 has a shoulder base part 27 provided on the distal side of the barrel part 22, and the tag mounting part 4. The tag mounting part 4 is composed of the covering part 5, the exposing part 6, and a shoulder body part 28.

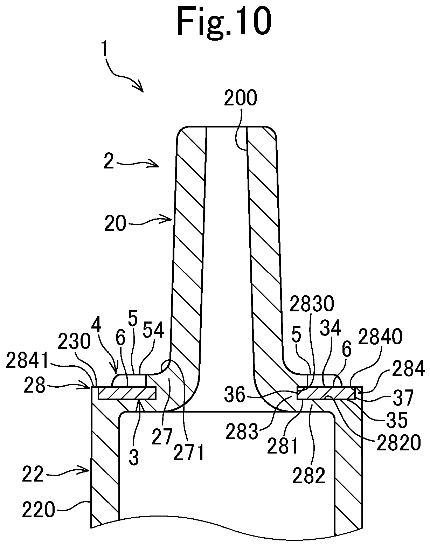

The shoulder base part 27 is an annular part extending from the distal end of the barrel part 22. The shoulder base part 27 extends from the distal edge of the barrel part 22 toward the distal side, and also extends radially outward. As shown in Fig. 10, a distal surface 271 of the shoulder base part 27 facing the distal side is a curved surface that is concave toward the proximal side. The distal surface 271 of the shoulder base part 27 can be a flat surface.

The shoulder body part 28 extends radially outward from the radially outer edge of the shoulder base part 27. The shoulder body part 28 is provided with a shoulder recess part 281 in which the wireless communication tag 3 is disposed. The shoulder body part 28 includes a shoulder support part 282 that supports the wireless communication tag 3. The shoulder body part 28 includes an inner side wall part 283 provided on the distal side of a part on the radially inner side of the shoulder support part 282, and an outer side wall part 284 provided on the distal side of a part on the radially outer side of the shoulder support part 282.

The shoulder support part 282 is a part that supports the wireless communication tag 3 from the proximal side. An edge 2821 on the radially outer side of the shoulder support part 282 is continuous with the outer surface 220 of the barrel part 22. The inner side wall part 283 is disposed on the radially inner side of the wireless communication tag 3. The outer side wall part 284 is disposed on the radially outer side of the wireless communication tag 3. An edge 2842 on the radially outer side of the outer side wall part 284 is continuous with an edge 2821 on the radially outer side of the shoulder support part 282. The shoulder recess part 281 is defined by a support surface 2820, which is a distal side surface of the shoulder support part 282, the edge 2830 on the radially outer side of the inner side wall part 283, and an edge 2840 on the radially inner side of the outer side wall part 284.

The covering part 5 is a part that partially covers the wireless communication tag 3 in the circumferential direction of the shoulder part 23 from the distal side (see Fig. 9). The covering part 5 is provided in continuous with the shoulder base part 27 and the shoulder body part 28. Four covering parts 5 are provided. In a configuration in which a plurality of the covering parts 5 are provided, it is preferable that at least one pair of covering parts 5 be disposed with the central axis of the shoulder part 23 therebetween.

In this pharmaceutical packaging container 1, the wireless communication tag 3 is mounted to the shoulder part 23 along the circumferential direction of the shoulder part 23. In this embodiment, the wireless communication tag 3 is disposed in the shoulder recess part 281 with its outer side partially covered by the covering parts 5. This configuration, in which the wireless communication tag 3 is disposed on the shoulder part 23, does not hinder the handling of the pharmaceutical packaging container 1.

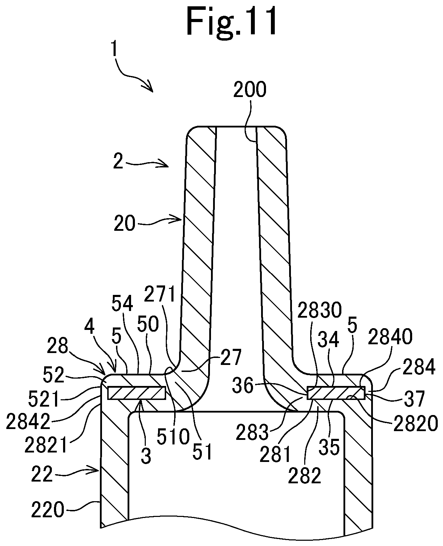

The cover inner edge part 51 is a part that connects the cover body part 50 and the shoulder base part 27 to each other (see Fig. 11). The distal surface 510 of the cover inner edge part 51 facing the distal side is continuous with the distal surface 271 of the shoulder base part 27.

The cover outer edge part 52 is a part that extends toward the radially outer side from the cover body part 50. The edge 521 on the radially outer side of the cover outer edge part 52 is continuous with the edge 2842 on the radially outer side of the outer side wall part 284 of the shoulder body part 28.

The exposing part 6 is a part that exposes the wireless communication tag 3 to the outer side from the outer surface 230 of the shoulder part 23 (see Fig. 10). In this embodiment, the exposing part 6 is a part that exposes the wireless communication tag 3 from a distal surface 2841, which is a surface of the outer side wall part 284 facing the distal side, toward the distal side.

The wireless communication tag 3 is sandwiched between the covering part 5 and the shoulder support part 282 of the shoulder body part 28 in the axial direction (see Fig. 11). The proximal surface 35, which is a main surface of the wireless communication tag 3 facing the proximal side, is in contact with the support surface 2820 of the shoulder support part 282.

The wireless communication tag 3 is sandwiched between the inner side wall part 283 and the outer side wall part 284 of the shoulder body part 28 in the radial direction. The edge 36 on the radially inner side of the wireless communication tag 3 is in contact with the edge 2830 on the radially outer side of the inner side wall part 283. The edge 37 on the radially outer side of the wireless communication tag 3 is in contact with the edge 2840 on the radially inner side of the outer side wall part 284.

In this embodiment, the distal surface 34 of the wireless communication tag 3 is flush with the distal surface 2841, which is a surface of the outer side wall part 284 facing the distal side (see Fig. 10). The distal surface 54 of the covering part 5 facing the distal side is flush with the distal surface 271 of the shoulder base part 27.

In this configuration, the flange part 24 is composed of the flange base part 25 and the flange body part 26 (see Fig. 8). In this embodiment, the outer edge 260 of the flange body part 26 has a circumferential shape. A distal surface 265, which is a surface facing the distal side of the flange body part 26, is a flat surface.

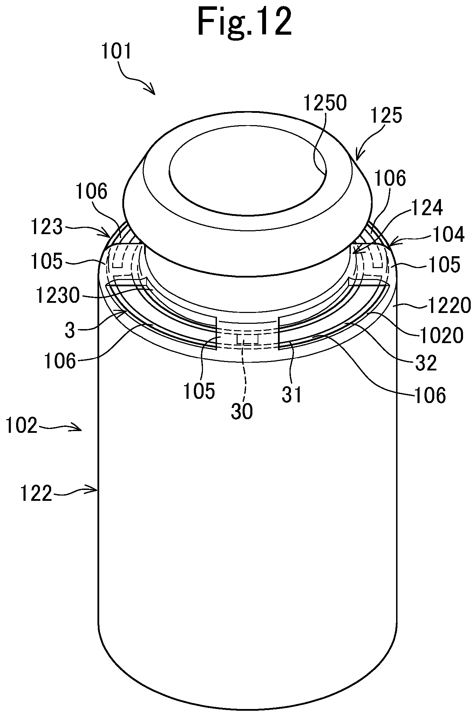

In the embodiments described above, the present invention is applied to the pharmaceutical packaging container 1 including the container body 2 with the openings 200 and 210 formed respectively at both end parts in the axial direction. However, the present invention can also be applied to a pharmaceutical container having a container body with an opening formed at one end part in the axial direction. A description will be hereinbelow given for an example of this configuration with reference to Fig. 12 to Fig. 15.

As shown in Fig. 12, a pharmaceutical container 101 includes a container body 102 and the wireless communication tag 3 mounted to the container body 102. The pharmaceutical container 101 in this embodiment is a vial, but it can also be a cartridge, a centrifuge tube, an ampoule, or the like.

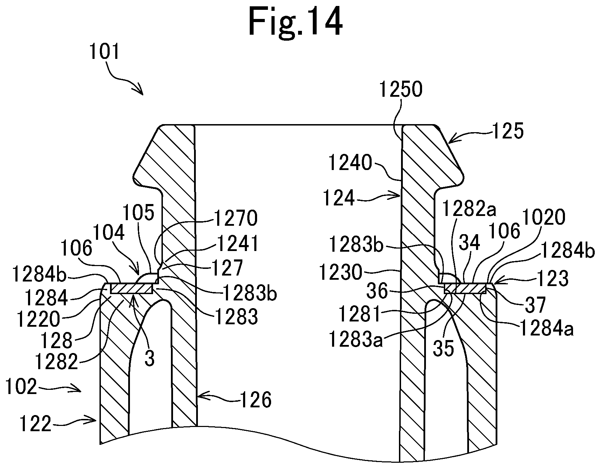

The container body 102 is capable of containing a medical preparation or pharmaceutical preparation inside. The container body 102 is made of synthetic resin. In this embodiment, the container body 102 includes a side wall part 122 having a cylinder shape, a shoulder part 123 extending radially inward from a distal end part 1220 in the axial direction of the side wall part 122, and a neck part 124 connected to an end part 1230 on the radially inward side of the shoulder part 123 and having a cylinder shape extending along the axial direction. As shown in Fig. 14, the container body 102 includes a rim part 125 that protrudes radially outward from the distal end part 1240 in the axial direction of the neck part 124, is formed in an annular shape around the axis, and has an opening 1250 on the radially inner side. The container body 102 includes an inner container part 126 having a bottomed cylinder shape disposed on the radially inner side of the side wall part 122. The container body 102 has a tag mounting part 104 for mounting the wireless communication tag 3 thereto.

The container body 102 contains liquid or solid medical or pharmaceutical preparation. In the pharmaceutical container 101, the side where the rim part 125 of the container body 102 is located, that is, the upper side in Fig. 12 is the "distal side," and the side where the side wall part 122 of the container body 102 is located, that is, the lower side in Fig. 12 is the "proximal side." The "axial direction" of the container body 102 is a cylinder axis direction of the side wall part 122. The radial direction orthogonal to the axial direction is also simply referred to as "radially."

The side wall part 122 has, for example, a cylindrical shape. The diameter of the side wall part 122 is uniform at all points in the axial direction, but can differ at least partly. For example, the side wall part 122 can have a tapered shape or a shape that bulges circumferentially at a central part in the axial direction. The side wall part 122 can have a rectangular tube shape. The continuous portion between the side wall part 122 and the shoulder part 123 has a slightly rounded shape, but it can also be an angular shape.

A plug member can be inserted into the opening 1250 of the rim part 125. The rim part 125 is engageable with a cover member that covers the plug member on its outer circumference. The rim part 125 is configured to allow a medical preparation or a pharmaceutical preparation to be put in and taken out of the pharmaceutical container 101 through the opening 1250.

The neck part 124 extends continuously from the rim part 125 toward the proximal side. The neck part 124 has, for example, a cylindrical shape. The outer diameter of the neck part 124 is smaller than the outer diameter of the rim part 125 and smaller than the outer diameter of the side wall part 122.

The inner container part 126 is a part for containing a medical preparation or pharmaceutical preparation (see Fig. 14). The inner container part 126 extends continuously from the end part 1230 on the radially inward side of the shoulder part 123 toward the proximal side. The inner container part 126 has a substantially cylindrical tube part located on the distal side of the inner container part 126, and a bottom portion that closes an opening on the proximal side of the tube part.

The shoulder part 123 is an annular part extending from the distal end part 1220 of the side wall part 122. Specifically, the shoulder part 123 extends from the distal end part 1220 of the side wall part 122 toward the distal side, and also extends radially inward. In this embodiment, the shoulder part 123 has a shoulder base part 127 provided on the proximal side of the neck part 124 and the tag mounting part 104.

The shoulder base part 127 is an annular part extending from a proximal end part 1241 of the neck part 124. Specifically, the shoulder base part 127 extends from the proximal end part 1241 of the neck part 124 toward the proximal side, and also extends radially outward. In this embodiment, a distal surface 1270 of the shoulder base part 127 facing the distal side is a curved surface that is recessed toward the proximal side. The distal surface 1270 of the shoulder base part 127 can be a flat surface.

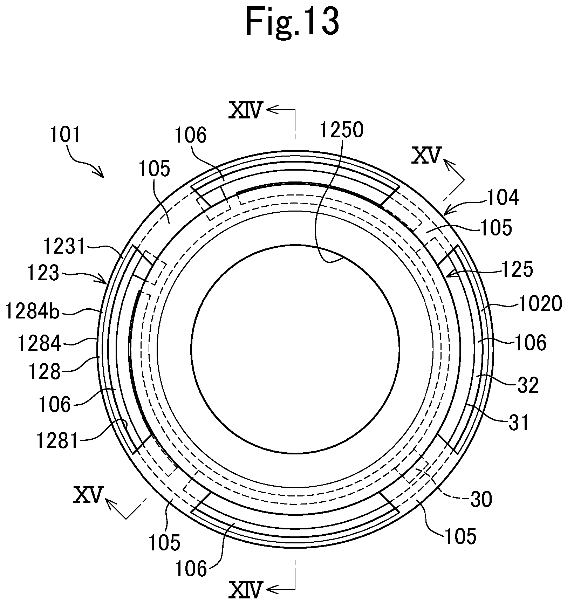

The tag mounting part 104 includes a covering part 105 that partially covers the wireless communication tag 3 from the outside, and an exposing part 106 that exposes the wireless communication tag 3 outward from the outer surface 1020 of the container body 102 (see Fig. 13). In this embodiment, the tag mounting part 104 is composed of a shoulder body part 128 in addition to the covering part 105 and the exposing part 106.

The shoulder body part 128 extends radially outward from the edge on the radially outer side of the shoulder base part 127 (see Fig. 14). The shoulder body part 128 is provided with a shoulder recess part 1281 in which the wireless communication tag 3 is disposed. The shoulder body part 128 includes a shoulder support part 1282 that supports the wireless communication tag 3. The shoulder body part 128 includes an inner side wall part 1283 provided on the distal side of a part on the radially inner side of the shoulder support part 1282, and an outer side wall part 1284 provided on the distal side of a part on the radially outer side of the shoulder support part 1282.

The shoulder support part 1282 is a part that supports the wireless communication tag 3 from the proximal side. The inner side wall part 1283 is disposed on the radially inner side of the wireless communication tag 3. The outer side wall part 1284 is disposed on the radially outer side of the wireless communication tag 3. An edge 1284c on the radially outer side of the outer side wall part 1284 is continuous with an edge 1282b on the radially outer side of the shoulder support part 1282. The shoulder recess part 1281 is defined by a support surface 1282a, which is a distal side surface of the shoulder support part 1282, an edge 1283a on the radially outer side of the inner side wall part 1283, and an edge 1284a on the radially inner side of the outer side wall part 1284.

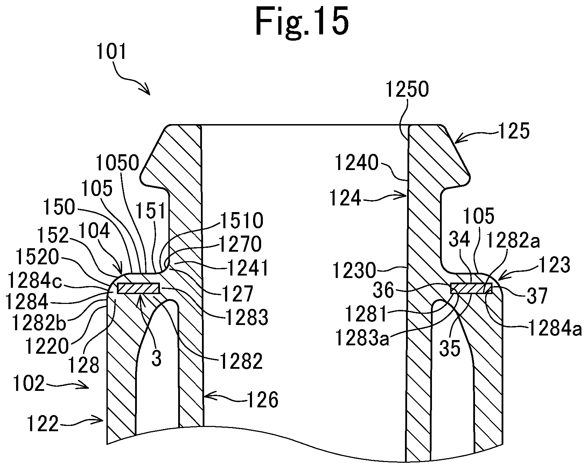

The covering part 105 is a part that covers the wireless communication tag 3 from the distal side, partially in the circumferential direction of the shoulder part 123 (see Fig. 13). The covering part 105 is provided in continuous with the shoulder body part 128 (see Fig. 15). The distal surface 1050 of the covering part 105 facing the distal side is a flat surface.

In this pharmaceutical packaging container 101, the wireless communication tag 3 is mounted to the shoulder part 123 along the circumferential direction of the shoulder part 123 (see Fig. 13). In the state where the wireless communication tag 3 is disposed in the shoulder recess part 1281, its outer surface is partially covered by the covering part 105. In this embodiment, the wireless communication tag 3 is mounted to the shoulder part 123 that connects the distal end part 1220 of the side wall part 122 and the proximal end part 1241 of the neck part 124 to each other (see Fig. 14).

A cover inner edge part 151 is a part that connects a cover body part 150 and the shoulder base part 127 to each other (see Fig. 15). The distal surface 1510 of the cover inner edge part 151 facing the distal side is continuous with the distal surface 1270 of the shoulder base part 127.

A cover outer edge part 152 is a part that extends radially outward from the cover body part 150. An edge 1520 on the radially outer side of the cover outer edge part 152 is continuous with the edge 1284c on the radially outer side of the outer side wall part 1284 of the shoulder body part 128.

The exposing part 106 is a part that exposes the wireless communication tag 3 outward from the outer surface 1231 of the shoulder part 123 (see Fig. 13). In this embodiment, the exposing part 106 is a part that exposes the wireless communication tag 3 toward the distal side from a distal surface 1284b, which is a surface of the outer side wall part 1284 facing toward the distal side.

The wireless communication tag 3 is sandwiched between the covering part 105 and the shoulder support part 1282 of the shoulder body part 128 in the axial direction (see Fig. 15). The proximal surface 35, which is a main surface of the wireless communication tag 3 facing the proximal side, is in contact with the support surface 1282a of the shoulder support part 1282.

The wireless communication tag 3 is sandwiched between the inner side wall part 1283 and the outer side wall part 1284 of the shoulder body part 128 in the radial direction. The edge 36 on the radially inner side of the wireless communication tag 3 is in contact with the edge 1283a on the radially outer side of the inner side wall part 1283. The edge 37 on the radially outer side of the wireless communication tag 3 is in contact with the edge 1284a on the radially inner side of the outer side wall part 1284.

In this embodiment, the distal surface 34 of the wireless communication tag 3 is flush with a distal surface 1283b, which is a surface of the inner side wall part 1283 facing the distal side (see Fig. 14). The distal surface 34 of the wireless communication tag 3 is flush with the distal surface 1284b, which is a surface of the outer side wall part 1284 facing the distal side. The distal surface 1050 of the covering part 105 is flush with the distal surface 1270 of the shoulder base part 127 (see Fig. 15).

In this pharmaceutical container 101, the wireless communication tag 3 is disposed on the container body 102 that contains the medical or pharmaceutical preparation, thereby ensuring traceability. Since the tag mounting part 104 includes the covering part 105 and the exposing part 106, the wireless communication tag 3 is fixed by the covering part 105, and the amount of resin used is reduced by an amount equivalent to expose the wireless communication tag 3 from the exposing part 106, thereby reducing the environmental impact.

The shoulder part 123 of this pharmaceutical container 101 is shaped to conform to the radial direction, but it can be a tapered shape, specifically a shape connected to the distal end part 1220 in the axial direction of the side wall part 122, while having an inner diameter becoming smaller toward the distal side in the axial direction. In this configuration, the neck part 124 is connected to the distal end part in the axial direction of the shoulder part 123.

The container body 102 of this pharmaceutical container 101 has a double structure including the side wall part 122 having a cylindrical shape and the inner container part 126 having a bottomed cylindrical shape disposed on the radially inner side of the side wall part 122, but it is not necessary to provide the inner container part 126. In this case, the container body 102 can be configured to include the side wall part 122 having a cylindrical shape and a bottom wall part that closes the opening on the proximal side of the side wall part 122.

It should be noted that the pharmaceutical packaging container and the pharmaceutical container of the present invention are not limited to the embodiments described above, and various modifications can be made without departing from the spirit of the present invention.

In the above embodiment, the covering part 5 covers the wireless communication tag 3 from the distal side, partially in the circumferential direction of the flange part 24 and the shoulder parts 23 and 123, but it can cover the wireless communication tag 3 partially in the radial direction of the flange part 24 or the shoulder parts 23 and 123. For example, the covering part 5 can be formed in an annular shape so as to circumferentially and continuously cover a central part of the wireless communication tag 3 in the radial direction.

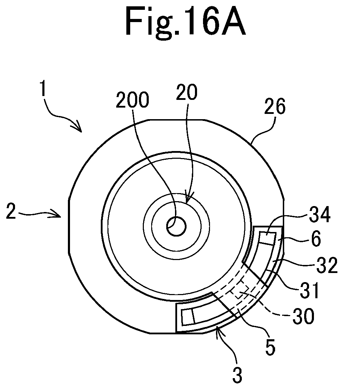

The wireless communication tag 3 has a substantially ring shape in the above embodiment, but it can have a circumferential shape with a plurality of corners, or, for example, an arc shape as shown in Fig. 16A to Fig. 16C. If the wireless communication tag 3 has an arcuate shape, the covering part 5 covers the wireless communication tag 3 partially in the direction in which the wireless communication tag extends, or covers the wireless communication tag 3 partially in the radial direction.

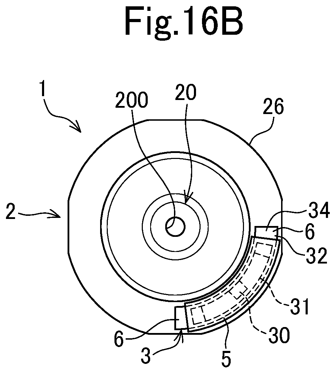

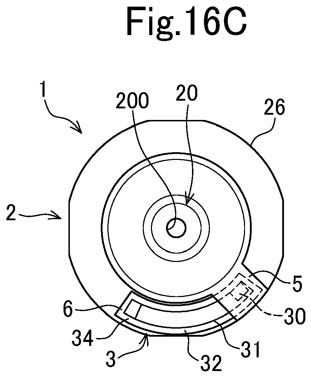

For example, the covering part 5 can cover a central part of the wireless communication tag 3 in the circumferential direction, while both end parts of the wireless communication tag 3 in the circumferential direction can be exposed from the exposing part 6 (see Fig. 16A). This exposing part 6 exposes most of the distal surface 34 of the wireless communication tag 3, specifically, exposes half or more of the area of the distal surface 34. The covering part 5 can cover most of the distal surface 34 of the wireless communication tag 3 (see Fig. 16B). Specifically, the covering part 5 can cover half or more of the area of the distal surface 34. The covering part 5 can cover one end part of the wireless communication tag 3 in the circumferential direction from the outside, while the central part and the other end part of the wireless communication tag 3 in the circumferential direction can be exposed from the exposing part 6 (see Fig. 16C).

In the above embodiment, one wireless communication tag 3 is provided for one pharmaceutical packaging container 1, but the configuration is not limited to this. For example, a plurality of wireless communication tags 3 can be provided for the pharmaceutical packaging container 1. In this case, the wireless communication tags 3 can be disposed with the central axis of the shoulder part 23 or flange part 24 of the pharmaceutical packaging container 1 therebetween. Alternatively, wireless communication tags 3 can be disposed one on each of the shoulder part 23 and the flange part 24 of the pharmaceutical packaging container 1. A plurality of wireless communication tags 3 can be provided for one pharmaceutical container 101.

In the above embodiment, the wireless communication tag 3 is mounted to the tag mounting part 4 by the hardening of the molten resin surrounding the wireless communication tag 3 during the injection molding process. However, the wireless communication tag 3 can be adhered to the tag mounting part 4 by applying a heat-sensitive adhesive or double-sided tape to the wireless communication tag 3. For example, a heat-sensitive adhesive or double-sided tape can be applied at least partly to each of surfaces 34, 35 and edges 36, 37 of the wireless communication tag 3 and then the wireless communication tag 3 is adhered to the tag mounting part 4.

According to the present invention thus described can provide a pharmaceutical packaging container and a pharmaceutical container that can reliably ensure traceability and reduce environmental impact.

A pharmaceutical packaging container including a container body that is made of synthetic resin, has an elongated shape, is capable of containing medicine inside, and has openings for inlet and outlet for the medicine formed respectively at a distal end part and a proximal end part in an axial direction of the container body, and a wireless communication tag mounted to an outer surface of the container body, the container body including a tag mounting part for mounting the wireless communication tag thereto, the tag mounting part including a covering part that partially covers the wireless communication tag from the outside, and an exposing part that exposes the wireless communication tag outward from the outer surface of the container body.

With this configuration, the wireless communication tag is disposed on the container body that contains the medicine inside, and therefore the traceability can be reliably ensured. Since the tag mounting part includes the covering part and the exposing part, the wireless communication tag is secured by the covering part, and the amount of resin used is reduced by an amount equivalent to expose the wireless communication tag, thereby reducing the environmental impact.

The pharmaceutical packaging container of the present invention can be configured such that the tag mounting part includes a plurality of the covering parts and a plurality of the exposing parts.

With this configuration, the wireless communication tag can be securely secured with the plurality of the covering parts, and the amount of resin used can be reduced by providing the plurality of the exposing parts.

The pharmaceutical packaging container can be configured such that the wireless communication tag includes a tag body that carries information, and an antenna part for allowing the tag body to communicate with an external device, the antenna part extending in a length direction that is a direction away from the tag body, the covering part configured to straddle the tag body or the antenna part in a direction intersecting the length direction.

With this configuration, the covering part straddles the outside of the wireless communication tag, ensuring secure fixation of the wireless communication tag. Furthermore, since the covering part extends in a direction intersecting the length direction of the antenna part, that is, the covering part covers the wireless communication tag partly in the length direction of the antenna part, the amount of resin used can be reduced.

The pharmaceutical packaging container of the present invention can be configured such that the wireless communication tag includes a tag body that carries information, and an antenna part for allowing the tag body to communicate with an external device, the antenna part extending in a length direction that is a direction away from the tag body, the plurality of covering parts configured to straddle the tag body or the antenna part in a direction intersecting the length direction.

With this configuration, the plurality of covering parts straddle the outside of the wireless communication tag, ensuring secure fixation of the wireless communication tag. Furthermore, since the plurality of covering parts extend in a direction intersecting the length direction of the antenna part, that is, the plurality of covering parts cover the wireless communication tag partly in the length direction of the antenna part, the amount of resin used can be reduced.

The pharmaceutical packaging container of the present invention can be configured such that the container body has a barrel part having a cylinder shape extending in the axial direction for containing medicine between the distal end part and the proximal end part, the proximal end part includes a flange part that is formed around the opening at the proximal end part while protruding from the outer surface of the barrel part in a radially outward direction orthogonal to the axial direction, and the wireless communication tag is mounted to the flange part along a circumferential direction of the flange part.

With this configuration, the wireless communication tag is mounted to the flange part along the circumferential direction of the flange part, thereby increasing the circumferential size of the wireless communication tag. This ensures that the communication range of the wireless communication tag can be maintained. By providing the wireless communication tag on the flange part, the wireless communication tag does not hinder the handling of the pharmaceutical packaging container. The visibility of the medicine contained in the container body is not impaired.

A pharmaceutical container of the present invention includes a container body that is made of synthetic resin, and capable of containing a medical or pharmaceutical preparation inside, the container body including: a side wall part having a cylinder shape; a shoulder part extending radially inward from a distal end part in an axial direction of the side wall part; a neck part connected to an end part on a radially inward side of the shoulder part and having a cylinder shape extending along the axial direction; and a rim part that protrudes radially outward from the distal end part in the axial direction of the neck part, is formed in an annular shape around the axis, has an opening on the radially inner side, is configured to allow a plug member to be fitted into the opening and a cover member for covering the plug member to be engaged with an outer circumference, the pharmaceutical container further including a wireless communication tag mounted to an outer surface of the container body, the container body including a tag mounting part to which the wireless communication tag is mounted, the tag mounting part including a covering part that partially covers the wireless communication tag from the outside and an exposing part that exposes the wireless communication tag outward from the outer surface of the container body.

With this configuration, the wireless communication tag is disposed on the container body that contains the medical or pharmaceutical preparation, thereby ensuring traceability. Since the tag mounting part includes the covering part and the exposing part, the wireless communication tag is fixed by the covering part, and the amount of resin used is reduced by an amount equivalent to expose the wireless communication tag from the exposing part, thereby reducing the environmental impact.

### REFERENCE SIGNS LIST

1: Pharmaceutical packaging container 2: Container body 3: Wireless communication tag 4: Tag mounting part 5: Covering part 6: Exposing part 7: Mold 20: Distal end part 21: Proximal end part 22: Barrel part 23: Shoulder part 24: Flange part 25: Flange base part 26: Flange body part 27: Shoulder base part 28: Shoulder body part 30: Tag body 31: Antenna part 32: Base part 34: Distal surface 35: Proximal surface 36, 37: Edge 50: Cover body part 51: Cover inner edge part 52: Cover outer edge part 53: Covering surface 54: Distal surface 70: Core: 71: Cavity 101: Pharmaceutical container 102: Container body 104: Tag mounting part 105: Covering part 106: Exposing part 122: Side wall part 123: Shoulder part 124: Neck part 125: Rim part 126: Inner container part 127: Shoulder base part 128: Shoulder body part 150: Cover body part 151: Cover inner edge part 152: Cover outer edge part 200, 210: Opening 220, 230, 240, 250: Outer surface 251: Distal surface 260: Outer edge 261: Flange recess part 262: Flange support part 263: Inner side wall part 264: Outer side wall part 265: Distal surface 271: Distal surface 281: Shoulder recess part 282: Shoulder support part 283: Inner side wall part 284: Outer side wall part 320: Outer edge 500: Distal surface 501: Proximal surface 510: Distal surface 521: Edge 700: Gate 701: Distal surface 710: Suction hole 711: Proximal surface 1020: Outer surface 1050: Distal surface 1220: Distal end part 1230: End part 1231: Outer surface 1240: Distal end part 1241: Proximal end part 1250: Opening 1270: Distal surface 1281: Shoulder recess part 1282: Shoulder support part 1282a: Support surface 1282b: Edge 1283: Inner side wall part 1283a: Edge 1283b: Distal surface 1284: Outer wall part 1284a: Edge 1284b: Distal surface 1284c: Edge 1510: Distal surface 1520: Edge 2600: Arcuate part 2601: Straight part 2620: Support surface 2621, 2630: Edge 2631: Distal surface 2640: Edge 2641: Distal surface 2642: Edge 2820: Support surface 2821: Edge 2830: Edge 2840: Edge 2841: Distal surface 901: Medical infusion device 902: Body 905: Hub portion 908: Needle 910: Cover 920: Outer casing 930: Inner casing 940: RFID tag

## Claims

1. A pharmaceutical packaging container comprising a container body that is made of synthetic resin, has an elongated shape, is capable of containing medicine inside, and has openings for inlet and outlet for the medicine formed respectively at a distal end part and a proximal end part in an axial direction of the container body, and
a wireless communication tag mounted to an outer surface of the container body,
the container body comprising a tag mounting part for mounting the wireless communication tag thereto,
the tag mounting part comprising a covering part that partially covers the wireless communication tag from the outside, and an exposing part that exposes the wireless communication tag outward from the outer surface of the container body.

2. The pharmaceutical packaging container according to claim 1, wherein the tag mounting part comprises a plurality of the covering parts and a plurality of the exposing parts.

3. The pharmaceutical packaging container according to claim 1, wherein the wireless communication tag includes a tag body that carries information, and an antenna part for allowing the tag body to communicate with an external device,
the antenna part extending in a length direction that is a direction away from the tag body,
the covering part configured to straddle the tag body or the antenna part in a direction intersecting the length direction.

4. The pharmaceutical packaging container according to claim 2, wherein the wireless communication tag includes a tag body that carries information, and an antenna part for allowing the tag body to communicate with an external device,
the antenna part extending in a length direction that is a direction away from the tag body,
the plurality of covering parts configured to straddle the tag body or the antenna part in a direction intersecting the length direction.

5. The pharmaceutical packaging container according to claim 1 or 2, wherein the container body has a barrel part having a cylinder shape extending in the axial direction for containing medicine between the distal end part and the proximal end part,
the proximal end part includes a flange part that is formed around the opening at the proximal end part while protruding from the outer surface of the barrel part in a radially outward direction orthogonal to the axial direction, and
the wireless communication tag is mounted to the flange part along a circumferential direction of the flange part.

6. A pharmaceutical container comprising a container body that is made of synthetic resin, and capable of containing a medical or pharmaceutical preparation inside,
the container body comprising: a side wall part having a cylinder shape;
a shoulder part extending radially inward from a distal end part in an axial direction of the side wall part;
a neck part connected to an end part on a radially inward side of the shoulder part and having a cylinder shape extending along the axial direction; and
a rim part that protrudes radially outward from the distal end part in the axial direction of the neck part, is formed in an annular shape around the axis, has an opening on the radially inner side, is configured to allow a plug member to be fitted into the opening and a cover member for covering the plug member to be engaged with an outer circumference,
the pharmaceutical container further comprising a wireless communication tag mounted to an outer surface of the container body,
the container body comprising a tag mounting part to which the wireless communication tag is mounted,
the tag mounting part comprising a covering part that partially covers the wireless communication tag from the outside and an exposing part that exposes the wireless communication tag outward from the outer surface of the container body.
